# EUROPEAN PATENT SPECIFICATION

(11) **EP 1 974 691 B1**
(45) Date of publication and mention of the grant of the patent: **25.05.2011**
(21) Application number: 07006420.9
(22) Date of filing: 28.03.2007
(51) Int. Cl.: A61C 19/06, A61L 2/20

(54) **Dental device**
Zahnmedizinische Vorrichtung
Dispositif dentaire

(43) Date of publication of application: 01.10.2008
(73) Proprietor: Krebber, Burghardt, 86899 Landsberg/Lech (DE)
(72) Inventor: Krebber, Burghardt, 86899 Landsberg/Lech (DE)
(74) Representative: Hartz, Nikolai

(56) References cited:
- DE-U1-202005 012 281
- US-A1- 2006 110 710

## Description

### Field of the Invention

The present invention relates a device for treating multiple teeth with a flow of ozone containing gas.

### Background of the invention

Decay of hard dental tissue is caused by microorganisms propagating in the vicinity of the hard dental tissue. Dental caries is based on the acid dissolution of enamel, dentine or cementum as a consequence of the metabolism of microorganisms living within deposits on the teeth known as plaque. Moreover, infections of soft dental tissue is caused by microorganisms propagating in the soft dental tissue.

In order to interrupt the progress of hard dental tissue decay, the decayed hard dental tissue including the microorganisms are commonly removed by a drilling treatment and the cavities thereby formed are sealed and filled with a restorative material. Dental treatment by drilling a tooth causes significant discomfort and anxiety to the patient. Moreover, the drilling treatment not only removes decayed dental tissue, but also healthy dental tissue. In addition, materials used for restorations may be problematic in that e.g. gold and ceramic are expensive and present a technical challenge for the practitioner whereas amalgam is potentially toxic and may cause allergic reactions. Therefore, alternative methods for the control of bacterial growth on and inside hard and soft dental tissue are desired.

In this context, the application of ozone to dental tissue was considered. WO02078644and US 2006/0110710 suggest the use of ozone in the treatment of dental and oral conditions. Ozone is a strongly oxidizing gas having bactericidal effects. However, ozone is also a highly toxic gas which may lead to severe poisoning when inhaled by a patient. Therefore, the application of ozone to hard and soft dental issue in the mouth of the patient requires applicator devices which are suitable for delivering a sufficiently high ozone concentration to the decaying dental tissue while at the same time avoiding contamination of the patient.

A dental applicator is known (OzonyTron commercially available from Mymed, Germany) which generates small amounts of ozone gas in a glass nozzle to be positioned in close proximity to the dental tissue to be treated. The ozone diffuses from an orifice of the nozzle so that a concentration gradient is formed wherein a bactericidally effective dose of ozone can only be expected within a distance of less than 3 mm from the orifice. According to this technology, a flow of ozone containing gas is not used. Given that only minor amounts of ozone are generated by the device and given that the treatment time does not exceed about 40 seconds, the risk of a contamination of the patient with ozone is believed to be acceptable despite the use of an open system. However, ozone is inevitably inhaled by the patient since means for eliminating gaseous ozone from the mouth of the patient are not provided. Furthermore, micro currents due to the ozone generation in a plasma inside the applicator nozzle placed in close proximity to the dental issue may be felt by the patient as an uncomfortable side effect. Finally, since the nozzle is made of a delicate glass structure, the device may break during the application of ozone.

A further dental applicator is known which uses a silicon cup placed on a dental surface to be treated. The silicon cup is connected to a vacuum pump for creating a vacuum inside a treatment chamber defined by the sleeve and the dental surface to be treated. The silicon cup forms a tight seal when a vacuum is applied. As soon as a predetermined vacuum is established, the device generates ozone and releases small amounts inside the treatment chamber for treatment of the dental surface. Ozone generation is discontinued when the pressure inside the silicon cup rises, indicating a leakage of the seal. Therefore, the performance of the applicator depends on the quality of the tight seal formed between the silicon cup and the dental surface to be treated. Given that decaying dental surfaces may not be able to form a tight seal due to the complicated surface topology, the tight seal may often not be reliably formed so that an application of ozone does not efficiently take place. As a remedy, a range of different silicone cups are provided which lead to further complications of the procedure.

Known dental applicators are not capable of administering ozone over an extended period of time so that a significant amount of ozone may be safely and efficiently delivered to decaying dental tissue in the treatment of deep caries.

Moreover, known dental applicators are not capable of treating a large dental surface over an extended period of time without the risk of contamination of the patient.

Finally, known dental applicators are not able of administering ozone over an extended period of time to soft tissue and saliva so that a significant amount of ozone may be safely and efficiently delivered and reach infected soft dental tissue by diffusion in the treatment of granuloma.

### Summary of the invention

It is the problem of the present invention to provide a device for safely and efficiently treating a hard and soft dental tissue with a flow of ozone.

It is a further problem of the present invention to provide a device for treating hard and soft dental tissue with a flow of ozone over an extended period of time with a device which may be easily handled and does not cause discomfort to the patient.

It is a further problem of the present invention to provide a device for treating a large surface of a hard and soft dental tissue or a dental surface which is difficult to access with a flow of ozone over an extended period of time.

The present invention provides a device for treating multiple teeth with a continuous flow of ozone containing gas, comprising
(a) an ozone generator for generating an ozone containing gas,
(b) a first pump being in fluid flow communication with the ozone generator for generating a flow of ozone containing gas,
(c) an applicator having a surface suitable for forming with multiple teeth to be treated a treatment chamber having an inlet orifice being in fluid flow communication with the ozon generator and the first pump and having an outlet orifice, and
(d) a second pump being in fluid flow communication with the outlet orifice of the applicator for removing gas from the treatment chamber,
whereby said second pump has a second throughput and the first pump has a first throughput whereby the ratio of the second throughput to the first throughput is at least 1.5 : 1.

The present invention is based on the recognition that dental caries and granuloma may be treated by exposing a dental tissue suffering from undesired bacterial growth to a continuous flow of ozone containing gas. In case of deep dental caries and granuloma, ozone may diffuse across plaque or tissue before reaching the microorganisms. Moreover, the present invention is based on the recognition that the success of a root canal treatment may be significantly increased when the root canal is treated with a flow of ozone containing gas.

The present invention is further based on the recognition that a continuous flow of ozone may be applied to a dental surface over an extended period of time without the risk of contamination of the patient when a combination of two pumps is used so that a first pump having a first throughput introduces an ozone containing gas into a treating chamber whereby a second pump having a higher throughput removes ozone containing gas from the treating chamber including air which has entered into the treating chamber through untight portions of the seal between the applicator and the dental tissue. The dental applicator forming the treating chamber may be provided by using conventional dental materials. The dental applicator may be adapted to the dentition of a specific patient for repeated application of ozone in the prevention and treatment of dental caries and granuloma.

The present invention uses a partial pressure of ozone in the ozone containing gas which is in a range previously unattainable when applying a vacuum and to dangerous to use in an open system. The high partial pressure of ozone in the continuous flow of oxygen as provided by the device of the present invention forces the ozone deep into the dental tissue whereby an efficient treatment of deep caries and granuloma is possible without the need for drilling treatment or surgery. Accordingly, it is possible to efficiently interrupt infection and decay of dental tissue due to bacterial growth while at the same time avoiding the pain and anxiety associated with a drilling treatment and surgery as well as avoiding unnecessary loss of healthy dental tissue or entire teeth.

### Brief Description of the Figures

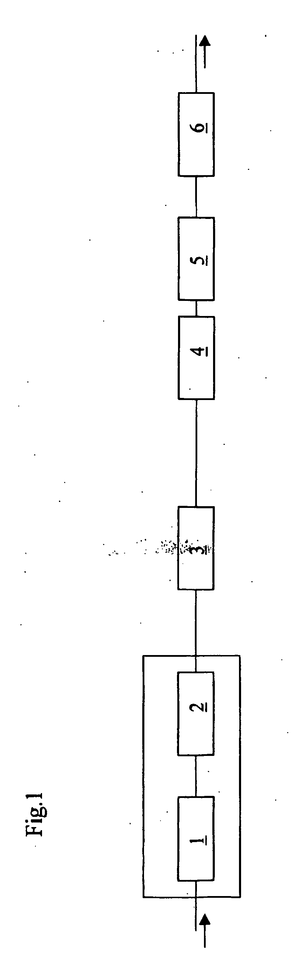

Figure 1 is a schematic representation of an embodiment of the device according to the invention.

### Description of the preferred embodiments

The present invention provides a device for treating a dental tissue with a continuous flow of ozone containing gas. The gas may be air having an oxygen content in the range of from 20 to 99 percent by volume. Accordingly, pure air may preferably be used or a mixture comprising air and additional pure oxygen gas.

The dental tissue to be treated includes hard and soft tissue. Hard tissue includes dentin and enamel as well as any restoration permanently provided on the dentin or enamel. Soft tissue includes any tissue in the mouth which is not hard tissue including the inside of pockets formed by the soft tissue. Treatment of dental tissue within the meaning of the present invention includes the exposure of dental surfaces to ozone molecules in a concentration suitable for controlling bacterial growth. Preferably, the treatment also includes the penetration of the dental surface by the ozone so that bacterial growth below the surface of the dental tissue may be controlled. Since anaerobic bacteria propagate in an environment where oxygen is not available, the treatment according to the present invention may be directed to providing an exposure of such bacteria even in such anaerobic environments. For this purpose, the dental tissue is exposed to a high ozone partial pressure over a prolonged period of time. Accordingly, it is possible to not only treat and prevent caries close to the surface, but also to control bacteria causing caries in layers below the dental tissue.

The device according to the invention comprises an ozone generator for generating an ozone containing gas. The ozone generator may be a conventional ozone generator using an electric discharge for cleaving oxygen molecules in a plasma. Preferably, the ozone generator allows to control the rate of ozone generation in order to adjust the ozone concentration in the ozone containing gas. The ozone containing gas generated by the ozone generator preferably has an ozone concentration of from 50 to 20.000 ppm, more preferably an ozone concentration of from 100 to 10.000 ppm. For safety reasons, the ozone generator is preferably arranged in series with the first and the second pump so that the ozone generator may only be activated when the pumps are running so that a flow of gas is present which prevents the ozone from being discharged from the ozone generator in an uncontrolled manner.

The device according to the present invention further comprises a first pump being in fluid flow communication with the ozone generator for generating a flow of ozone containing gas. Preferably, the first pump is arranged so as to discharge a stream of gas such as air into the ozone generator for providing a continuous flow of ozone containing gas downstream from the first pump. The first pump has a first throughput. Moreover, the first pump has a first pumping speed. Preferably, the first pump has a pumping speed in the range of from 1 to 10 I/min, more preferably from 2 to 6 I/min.

The device according to the present invention further comprises an applicator having a surface suitable for forming with multiple teeth to be treated a treatment chamber having an inlet orifice being in fluid flow communication with the ozon generator and the first pump and having an outlet orifice. The applicator may be adapted for the treatment of different types of dental tissues.

The applicator may be designed so as to fit a number of different patients. Accordingly, an applicator may be provided which consists of sterilizable material.

In a further preferred embodiment, the applicator according to the present invention is a custom made applicator which is adapted to the teeth of a specific patient. Accordingly, the applicator may be obtainable by a process comprising
(i) providing a dental cast of a dentition;
(ii) adding material to the cast surface corresponding to the surface to be treated;
(iii) shaping the material so as to provide a continuous body corresponding to the shape of a treatment chamber;
(iv) providing a curable material on the outer surface of the material bearing cast so as to provide an applicator having a surface suitable for forming with the dental tissue to be treated a treatment chamber.

In an example, which does not form part of the present invention, the applicator is an applicator for a single tooth, preferably for a prepared root canal of a single tooth. Accordingly, the applicator may be a adapted to cover the cavity. The inlet orifice being in fluid flow communication with the ozone generator and the first pump may be provided at the distal end of a nozzle which may be introduce deeply into the root canal. Moreover, the outlet orifice may be provided so that an efficient flow of ozone containing gas may be established preferably from the apex of the root canal across the entire length of the root canal so as to increase the efficiency of the treatment. The nozzle may be made of a flexible material so that breaking of the nozzle in the root canal may be avoided. Accordingly, the applicator may be an applicator for a root canal of a single tooth, which comprises a flexible hollow needle suitable for being inserted into a root canal.

The device according to the present invention further comprises a second pump being in fluid flow communication with the outlet orifice of the applicator for removing gas from the treatment chamber. The second pump has a second throughput. The ratio of the second throughput to the first throughput is at least 1.5 : 1, preferably 2:1. Moreover, the second pump preferably has a pumping speed in the range of from 1.5 to 15 l/min, more preferably in the range of from 2 to 10 I/min. Preferably, the activation of the second pump is a necessary condition for the possibility of activating the ozone generator so that the contamination of the patient may be excluded.

The device of the present invention may further comprise an ozone scavenging means which is in fluid communication with the outlet orifice of the applicator. The scavenging means may contain a reducing agent which eliminates any ozone remaining in the flow of ozone containing gas discharged from the treatment chamber before entering the second pump.

Moreover, the device of the present invention may further comprise a moisture trapping means which is in fluid communication with the outlet orifice of the applicator. The moisture trapping means may contain a drying agent or a cold surface for eliminating any water vapors remaining in the flow of ozone containing gas discharged from the treatment chamber before entering the second pump.

The device according to the present invention may be part of a system comprising a device for treating a dental tissue with a continuous flow of ozone containing gas, and a plurality of applicators.

The device for treating a dental tissue with a continuous flow of ozone containing gas may be used as follows. Prior to activating the ozone generator, the applicator is placed in the patients mouth so that a treating chamber is formed by the inner surface of the applicator and the dental tissue to be treated. Subsequently, a gas flow may be initiated by activating the first pump and the second pump. Provided that the gas flow is established, the ozone generator may be activated. Accordingly, the flow of gas will then include an amount of ozone to which the dental surface inside the treating chamber is exposed. The treatment of a dental tissue of a patient by applying a continuous flow of ozone for a period of time may be applied for a predetermined time in the range of from 30 seconds to 60 minutes.

Figure 1 shows a schematic representation of a preferred embodiment of the device according to the invention. A first pump 1 is fluid communication with an ozone generator. The first pump 1 and the ozone generator 2 may be combined in a single device for providing a flow of ozone gas. The ozone generator 3 is in fluid flow communication with the dental applicator 3. The dental applicator is in fluid flow communication with a moisture trapping means 4 which dries the flow of gas from the dental device. The moisture trapping means 4 is in fluid flow communication with a ozone scavenging means 5 for eliminating excess ozone present in the flow of gas. The ozone scavenging means 5 is in fluid flow communication with a second pump 5 which is a suction pump. The order of the moisture trapping means and the ozone scavenging means may be reversed.

The preparation of a preferred dental applicator according to the present invention will now be described. In a first step, a conventional dental cast is provided. The dental cast may be based on an impression of the relevant portion of the dentition of a specific patient. The dental cast may also be a standard cast of a size which fits a number of different patients. The cast may be made of a conventional dental material such as gypsum.

In a next step, the portions of the dental cast corresponding to the dental tissue to be exposed to a flow of ozone gas is covered with a layer of a thermoplastic material such as a dental wax. The wax material will define the inner volume of the treatment chamber in the final dental applicator. The layer of wax is then covered with a layer of light-curing resin for defining the walls of the dental applicator which will define the treatment chamber in the final dental applicator. An orifice is provided for the connectors of tubing directing the flow of ozone to the treatment chamber of the dental applicator. Advantageously, the connectors are provided at the anterior portion of the dental applicator so that the tubing will not extend into the mouth of the patient during treatment. Subsequently, the resin is cured whereby the connector is integrated into the resin structure. Orifices are provided at the upper side of the resin material, preferably in a posterior position, which serve as exits for the flow of ozone gas from the treatment chamber into a discharge channel guiding the flow of gas to a discharge orifice of the dental applicator at the anterior portion of the applicator. The discharge channel is then provided as follows. After the curing of the first resin layer, a second wax layer is provided on the cured resin layer which defines the volume of the discharge channel. The wax layer covers the orifices provided in the first resin layer. The wax layer is provided so that a contact between the subsequently applied resin with the first resin layer is still possible along the circumference of the dental applicator. Subsequently, a second resin layer is provided on the second wax layer so that a second chamber is formed on top of the first chamber. A further connector for tubing is also provided which will direct the flow of gas out of the dental applicator. The second resin layer will now be cured so that the second resin layer will be attached to the first resin layer and defined a discharge channel. In the next step, the thermoplastic material will be molten so that the flow chambers are formed. Along the lower edges of the applicator, retentions are provided preferably by drilling. In these retentions and beyond the lower edges, walls are provided with wax corresponding to the portion of the final dental applicator which extends to the soft dental tissue thereby sealing the treatment chamber. The wax walls are covered in conventional manner with gypsum or hard silicon to provide mold faces. The wax will then be removed by heating and the connecting surfaces are cleaned and roughened and provided with a silicon bonding material. Subsequently, the device is placed on the dental cast and the outer and inner mold faces are attached. On the both sides of the mold, a hole will be drilled and a chamber will now be filled with silicon. The silicone is subsequently cured. After curing, the applicator may be used.

Now the use of the applicator will be described which does not form part of the claimed invention. The applicator is placed in the mouth of the patient and both connectors are connected to tubing so that the inner chamber forming a treatment chamber with the dental tissue is connected to the ozone generator and the connector of the discharge channel is connected to the tubing for fluid flow communication with the second pump. Now the second pump is activated so that gas is removed by suction from the dental device. Subsequently, the first pump and the ozone generator are activated whereby a flow of ozone containing gas enters the treatment chamber and exits through the second chamber of the dental applicator. Since the through-put of the second pump is higher than the through-put of the first pump, ozone gas cannot be inhaled by the patient.

## Claims

1. A device for treating multiple teeth with a continuous flow of ozone containing gas, comprising
(a) an ozone generator (2) for generating an ozone containing gas,
(b) a first pump (1) being in fluid flow communication with the ozone generator for generating a flow of ozone containing gas,
(c) an applicator (3) having a surface suitable for forming with multiple teeth to be treated a treatment chamber having an inlet orifice being in fluid flow communication with the ozone generator (2) and the first pump (1) and having an outlet orifice, and
(d) a second pump (6) being in fluid flow communication with the outlet orifice of the applicator for removing gas from the treatment chamber,
whereby said second pump has a second throughput and the first pump has a first throughput whereby the ratio of the second throughput to the first throughput is at least 1.5:1.

2. The device according to any one of the preceding claims, wherein the ozone containing gas generated by the ozone generator has an ozone concentration of from 50 to 20.000 ppm.

3. The device according to any one of the preceding claims wherein the gas is air having an oxygen content in the range of from 20 to 99 percent by volume.

4. The device according to any one of the preceding claims, wherein the first pump has a pumping speed in the range of from 1 to 10 l/min.

5. The device according to any of the preceding claims wherein the second pump has a pumping speed in the range of from 1.5 to 15 l/min.

6. The device according to any one of the preceding claims which further comprises an ozone scavenging means downstream from the outlet orifice of the applicator and upstream from the second pump.

7. The device according to any one of the preceding claims which further comprises a moisture trapping means downstream from the outlet orifice of the applicator and upstream from the second pump.

8. A system comprising a device according to any one of claims 1 to 7, and a plurality of applicators.

9. The system according to claim 8 wherein the applicator is for the treatment of multiple adjacent teeth, which is obtainable by a process comprising
(i) providing a dental cast of a dentition;
(ii) adding material to the cast surface corresponding to the surface to be treated;
(iii) shaping the material so as to provide a continuous body corresponding to the shape of a treatment chamber,
(iv) providing a curable material on the outer surface of the material bearing cast so as to provide an applicator having a surface suitable for forming with the dental tissue to be treated a treatment chamber.

## Patentansprüche

1. Vorrichtung zur Behandlung mehrerer Zähne mit einem kontinuierlichen Strom eines Ozon enthaltenden Gases, umfassend
(a) einen Ozongenerator (2) zur Erzeugung eines Ozon enthaltenden Gases,
(b) eine erste Pumpe (1), die in Fließverbindung mit dem Ozongenerator steht zur Erzeugung eines Stroms von Ozon enthaltendem Gas,
(c) einen Applikator (3) mit einer Oberfläche, die geeignet ist mit mehreren Zähnen, die behandelt werden sollen, eine Behandlungskammer zu bilden, die eine Einlassöffnung aufweist, die in Fließverbindung mit dem Ozongenerator (2) steht sowie mit der ersten Pumpe (1) und die eine Auslassöffnung aufweist, und
(d) eine zweite Pumpe (6), die in Fließverbindung steht mit der Auslassöffnung des Applikators, um Gas aus der Behandlungskammer zu entfernen,
wobei die zweite Pumpe einen zweiten Durchsatz und die erste Pumpe einen ersten Durchsatz aufweist, wobei das Verhältnis des zweiten Durchsatzes zu dem ersten Durchsatz mindestens 1,5 : 1 beträgt.

2. Die Vorrichtung nach Anspruch 1, wobei das Ozon enthaltende Gas, das von dem Ozongenerator erzeugt wird eine Ozonkonzentration im Bereich von 50 bis 20.000 ppm aufweist.

3. Die Vorrichtung nach einem der vorstehenden Ansprüche, wobei das Gas Luft ist, die einen Sauerstoffgehalt im Bereich von 20 bis 99 Volumenprozent aufweist.

4. Die Vorrichtung nach einem der vorstehenden Ansprüche, wobei die erste Pumpe eine Pumpgeschwindigkeit im Bereich von 1 bis 10 l/min aufweist.

5. Die Vorrichtung nach einem der vorstehenden Ansprüche, wobei die zweite Pumpe eine Pumpgeschwindigkeit im Bereich von 1,5 bis 15 l/min aufweist.

6. Die Vorrichtung nach einem der vorstehenden Ansprüche, die ferner ein Ozonauffangmittel umfasst, das sich stromab von der Auslassöffnung des Applikators und stromauf von der zweiten Pumpe befindet.

7. Die Vorrichtung nach einem der vorstehenden Ansprüche, die ferner ein Feuchtigkeitsauffangmittel umfasst, das stromab von der Auslassöffnung des Applikators und stromauf von der zweiten Pumpe angeordnet ist.

8. System, umfassend eine Vorrichtung nach einem der Ansprüche 1 bis 7 sowie eine Mehrzahl von Applikatoren.

9. Das System nach Anspruche 8, wobei der Applikator zur Behandlung von einer Mehrzahl benachbarter Zähne geeignet und erhältlich ist durch ein Verfahren umfassend
(i) Bereitstellung eines Dentalmodells eines Gebisses;
(ii) Zugabe eines Materials auf die Modelloberfläche, die der zu behandelnden Oberfläche entspricht;
(iii) Formen des Materials, um einen kontinuierlichen Körper zu bilden, der der Form einer Behandlungskammer entspricht;
(iv) Bereitstellung eines härtbaren Materials auf der äußeren Oberfläche des das Material tragenden Modells, um einen Applikator zu schaffen, der eine Oberfläche aufweist, die geeignet ist zur Bildung einer Behandlungskammer mit dem Dentalgewebe, das zu behandeln ist.

## Revendications

1. Dispositif pour traiter plusieurs dents avec un flux continu de gaz contenant de l'ozone, comprenant
(a) un générateur d'ozone (2) pour générer un gaz contenant de l'ozone,
(b) une première pompe (1) qui est en communication fluidique avec le générateur d'ozone pour générer un flux de gaz contenant de l'ozone,
(c) un applicateur (3) qui présente une surface apte à former avec plusieurs dents à traiter une chambre de traitement pourvue d'un orifice d'admission en communication fluidique avec le générateur d'ozone (2) et la première pompe (1), et pourvue d'un orifice de sortie, et
(d) une seconde pompe (6) qui est en communication fluidique avec l'orifice de sortie de l'applicateur, pour évacuer le gaz de la chambre de traitement,
étant précisé que la seconde pompe a un second débit et la première pompe un premier débit, et que le rapport du second débit sur le premier débit est d'au moins 1,5:1.

2. Dispositif selon l'une quelconque des revendications précédentes, dans lequel le gaz contenant de l'ozone qui est généré par le générateur d'ozone a une concentration en ozone de 50 à 20.000 ppm.

3. Dispositif selon l'une quelconque des revendications précédentes, dans lequel le gaz est constitué par de l'air présentant une teneur en oxygène située dans la plage de 20 à 99 pour cent en volume.

4. Dispositif selon l'une quelconque des revendications précédentes, dans lequel la première pompe a une vitesse de pompage située dans la plage de 1 à 10 l/min.

5. Dispositif selon l'une quelconque des revendications précédentes, dans lequel la seconde pompe a une vitesse de pompage située dans la plage de 1,5 à 15 l/min.

6. Dispositif selon l'une quelconque des revendications précédentes, qui comprend également des moyens d'expulsion d'ozone en aval de l'orifice de sortie de l'applicateur et en amont de la seconde pompe.

7. Dispositif selon l'une quelconque des revendications précédentes, qui comprend également des moyens de séparation d'humidité en aval de l'orifice de sortie de l'applicateur et en amont de la seconde pompe.

8. Système comprenant un dispositif selon l'une quelconque des revendications 1 à 7, et plusieurs applicateurs.

9. Système selon la revendication 8, dans lequel l'applicateur est destiné au traitement de plusieurs dents voisines, que l'on peut obtenir à l'aide d'un procédé comprenant les étapes qui consistent:
(i) à prévoir un moulage dentaire pour une dentition;
(ii) à ajouter un matériau à la surface de moulage correspondant à la surface à traiter;
(iii) à former le matériau de manière à donner un corps continu correspondant à la forme d'une chambre de traitement;
(iv) à prévoir un matériau durcissable, sur la surface extérieure du moulage portant le matériau, de manière à donner un applicateur avec une surface apte à former avec le tissu dentaire à traiter une chambre de traitement.
